# EUROPÄISCHE PATENTSCHRIFT

(11) **EP 1 296 909 B1**
(45) Veröffentlichungstag und Bekanntmachung des Hinweises auf die Patenterteilung: **07.06.2006**
(21) Anmeldenummer: 01957914.3
(22) Anmeldetag: 03.07.2001
(51) Int. Cl.: C04B 28/34, A61L 24/02, A61K 6/033

(54) **MAGNESIUM-AMMONIUM-PHOSPHAT-ZEMENTE, DEREN HERSTELLUNG UND VERWENDUNG**
MAGNESIUM-AMMONIUM-PHOSPHATE CEMENTS, THE PRODUCTION OF THE SAME AND THE USE THEREOF
CIMENTS PHOSPHATE DE MAGNESIUM-AMMONIUM, LEUR PREPARATION ET LEUR UTILISATION

(30) Priorität: 03.07.2000 DE 10032220
(43) Veröffentlichungstag der Anmeldung: 02.04.2003
(73) Patentinhaber: Sanatis GmbH, 01454 Radeberg (DE)
(72) Erfinder: ZIMMERMANN, Michael, 65931 Frankfurt am Main (DE)
(74) Vertreter: Hofer, Dorothea
(86) Internationale Anmeldenummer: PCT/EP2001/007605
(87) Internationale Veröffentlichungsnummer: WO 2002/002478

(56) Entgegenhaltungen:
- EP-A- 0 543 765
- WO-A-95/13835
- US-A- 4 612 053
- DATABASE CA [Online] CHEMICAL ABSTRACTS SERVICE, COLUMBUS, OHIO, US; PARK, SANG-JONG ET AL: "Compositional effects of CaO-SiO2-P2O5 bioactive cement on hardening and hydroxyapatite formation" retrieved from STN Database accession no. 121:141597 CA XP002180738 & YOOP HAKHOECHI (1994), 31(5), 502-12 , 1994,
- DATABASE WPI Section Ch, Week 199204 Derwent Publications Ltd., London, GB; Class D22, AN 1992-027364 XP002180739 & JP 03 272771 A (NIPPON ELECTRIC GLASS CO), 4. Dezember 1991 (1991-12-04)

## Beschreibung

Die Erfindung betrifft eine Magnesium-ammonium-phosphat-Zement-Zubereitung, deren Verwendung zur Herstellung eines Magnesium-ammonium-phosphat-Zements, sowie deren Verwendung für andere zwecke.

Diese Erfindung betrifft insbesondere einen biologisch abbaubaren Zement, der nach Aushärtung *in seiner Hauptphase* aus Magnesium-ammonium-phosphaten und Nanoapatiten besteht und dabei gleichzeitig eine hohe Festigkeit aufweist.
Das Material kann als Knochenersatz, zur Knochenaugmentation und zur Knochenregeneration eingesetzt werden.
Es kann als Trägermaterial für pharmazeutische oder biologische Wirkstoffe dienen.

Die wichtigsten Mineralbestandteile im menschlichen Knochen und Zahnhartgewebe sind Calcium und Phosphat. Aber auch beachtliche Mengen von Natrium, Magnesium und Carbonat sind vorhanden.
Aus Präzipitationsstudien von synthetischen Systemen weiß man, daß Natriumionen und Carbonationen sehr leicht in Calcium-phosphat-präzipitate inkorporiert werden können mit dem Ergebnis einer apatitähnlichen Molekülstruktur.
Allerdings hat Magnesium eine starke Neigung zur Fällung in einer anderen, nicht apatitähnlichen Struktur.

Physiologisch als Knochen und Dentin präzipitiertes Calciumphosphat ist nanokristallin. Im Röntgendiffraktogramm ist wegen der Linienverbreiterungen nicht erkennbar, ob es sich dabei um Apatit oder um andere Strukturen handelt.
Einige Wissenschaftler sind der Meinung, daß in Knochen und Dentin so viel Magnesium vorkommt, daß dies nicht alles in der Apatitstruktur aufgenommen werden kann. Deshalb vermutet man hier eine Mischform des Minerals aus Nanoapatit und Nanodolomit bzw. Nanostruvit.
Calciumphosphate sind nicht nur biokompatibel, sondern werden von der lebenden Zelle als körperzugehörig erkannt. Deshalb gibt es viele Biomaterialien und Medizinprodukte, die teilweise aus Calciumphosphat bestehen.

Seit ca. 1970 gibt es Calciumphosphat - Keramiken auf dem Markt, teils in Form von vorgefertigten Blocks oder als Granulate. Implantationen von diesen Materialien in Knochenstrukturen sind überwiegend erfolgreich.
Der größte Nachteil dieser Systeme ist, daß die Blocks vorgefertigt sein müssen und die Granulate von der Implantationsseite wegdriften (Ausschwemmen). Dies führt oft zum Scheitern solcher Implantationen.

Calciumphosphat - Keramiken sind höchst erfolgreich, wenn sie aus Hydroxyl-apatit (HA) oder aus beta-tertiären Calciumphosphat (β-TCP, eine whitloctit-artige Struktur) bestehen oder wenn die Calciumphosphat - Keramiken aus beidem, HA und β-TCP in variablen Verhältnissen bestehen.
Aus Knochenimplantationen ist HA praktisch nicht resorbierbar, wohingegen β-TCP langsam resorbiert und durch neuen Knochen ersetzt wird.
Es ist daher möglich, durch Verändern des β-TCP/HA - Verhältnisses den Grad der Resorption der Calciumphosphat - Keramik zu beeinflussen.
Es ist ebenso möglich, andere resorbierbare Stoffe beizumischen wie: Monetit CaHPO₄, Brushit CaHPO₄-2H₂O, Calcit CaCO₃ und Dolomit CaMg(CO₃)₂.

Seit 1985 versucht man, Calciumphosphat-Zemente zu entwickeln, um die Nachteile von vorgefertigten oder granulatförmigen Calciumphosphat-Keramiken zu umgehen (W.E. Brown and L.C. Chow, "A new calcium phosphate, water - setting cement", Cem. Res. Prog. 1986 352-379(1987)).
Darunter ist ein noch nicht im Handel befindlicher Brushit - Zement mit einem Ca/P Molverhältnis der gefällten Phase von 1,00. Diese Phase ist nicht nanokristallin, sondem mikrokristallin.
Alle anderen bisher entwickelten Calciumphosphat-Zemente besitzen eine nanokristalline Fällungsstruktur und ein Ca/P Molverhältnis von ≥ 1,5 das durch Zusatz von Carbonat noch vergrößert werden kann. Diese Materialien sind unter U.S. 5,605,713; EP 0 835 668 A1; WO 96/14265; bekannt, und manche dieser Materialien sind bereits auf dem Markt. Bezüglich der Resorbierbarkeit dieser Materialien nach Implantationen in Knochen und weichem Gewebe gibt es widersprüchliche Berichte.
In jedem Fall unterscheidet man Calciumphosphat-Zemente, aufbauend auf Hydroxylapatit (HA), die nicht resorbierbar sind (HA Keramiken s.o.), und Calciumphosphat-Zemente, aufbauend auf defizienten Calciumhydroxylapatiten (CDHA, Calcium deficient Hydroxyapatite), die gut osteotransductiv sind.
Das bedeutet für die Letztgenannten, daß sie von Osteoklasten resorbiert und von Osteoplasten durch neues Knochengewebe ersetzt werden können.
Die Resorption dieser Zemente hängt entscheidend von den lokalen Knochenumbaumechanismen ab.
Heutzutage wird von den meisten Chirurgen ein Calciumphosphat-Zement gewünscht, bei dem zunächst eine mechanisch unterstützende Wirkweise zum Tragen kommt, aber die letztendliche Resorption unabhängig von den lokalen Umbaumechanismen des Knochens abhängt, d. h. daß das Material vollständig abgebaut wird. Außerdem ist in der Orthopädie bekannt, daß vitaler Knochen nur dort verbleibt, wo er aus biomechanischer Sicht benötigt wird. Dies ist als sogenanntes Wofff sches Gesetz bekannt. Hat demzufolge ein in einen Kochendefekt eingebrachter Calziumphosphatzement eine höhere Druckfestigkeit als der ihn umgebende Knochen und bleibt diese hohe Druckfestigkeit unverändert erhalten, so führt dies zum Abbau von um das Implantat (hier Calzoumphosphatzement) liegendem Knochengewebe.

Um diese Forderung wenn auch nur teilweise zu erfüllen, haben manche Hersteller Substanzen in ihre nanoapatit-ähnlichen CDHA-Zemente beigemischt, die von den Körperflüssigkeiten aufgrund der Konzentrationsgradienten passiv resorbiert werden, wie z.B. Monetit (CaHPO₄) oder Calcit (CaCO₃), wie aus EP 0 543 765 bekannt.

Dies löst das Problem aber nur teilweise. Es wird weiterhin ein Zement benötigt, der vollständig passiv resorbierbar ist und bei dem die Resorptionsfront und die Ablagerungsfront in direktem Kontakt liegen.
Gips beispielsweise erfüllt diese Forderung nicht Gips ist so schnell resorbiert, daß immer eine Lücke zwischen der Resorptionsfront und der Ablagerungsfront klafft und diese Materialien aufgrund ihrer niedrigen Druckstabilität keine ausreichende unterstützende Funktion besitzen. Solche Materialien sind beispielsweise unter U.S. 5,281,265 offenbart.

Es ist aus diesen Gründen wünschenswert, ein Knochenersatzmaterial zur Verfügung zu stellen, welches zunächst mit hoher Druckstabilität die verlorengegangene Stützfunktiori des Knochens übernimmt, dann aber sukzessive an Druckstabilität abnimmt, wodurch die körpereigenen Knochenumbauvorgänge (Remodelling) angeregt werden und somit eine schnellere Osteoneogenese und damit auch aktive Resorption des Knochenersatzmaterials eingeleitet wird. Dies kann auch dadurch erreicht werden, indem z.B. in eine aushärtende Zementpaste eine leicht lösliche Substanz eingearbeitet wird. Weil Knochen gut in makroporöse Strukturen einwächst, ist es vorteilhaft, granuläre oder pelletförmige, solubilisierende Substanzen bestehend aus z.B. Zuckern, Salzen (z.B. NaCl) oder Gips (CaSO₄) in die Zementpaste einzumischen. Diese werden dann sehr schnell im Körper aus dem gehärteten Zementgerüst herausgelöst, und übrig bleibt eine poröse schwammartige Struktur. Denkbar ist auch eine Herstellung der porösen (fertigen) Zemente außerhalb des Körpers.

Um einen Zement für dentale Anwendungen, wie z. B. Füllung und Verschluß von Dentinkanälchen, Füllung von Zahnhöhlen nach Vitalextirpation, Benutzung eines solchen Zementes als Unterfüllungswerkstoff in der Endodontologie, benutzen zu können, darf ein solches Material nicht schrumpfen, um einen Durchtritt von Bakterien zu verhindern. Wünschenswert wäre sogar ein Material mit geringgradig expandierbaren Eigenschaften.

Es ist Aufgabe der Erfindung, eine Zement-Zubereitung zur Verfügung zu stellen, mit der die Nachteile des Standes der Technik vermieden werden.

Die Aufgabe wird gelöst durch eine Magnesium-ammonium-phosphat-Zement-Zubereitung nach den Ansprüchen 1, 13 und 15, durch ein Verfahren zur Herstellung einer Magnesium-ammonium-phosphat-Zements unter Verwendung der Zubereitung nach Anspruch 23 und durch die Verwendung nach den Ansprüchen 26 bis 28. Weiterbildungen der Erfindung sind in den Unteransprüchen angegeben.

Die Probleme beim Stand der Technik werden durch vorliegende Erfindung bevorzugt dadurch gelöst, daß durch Variation in der Zumischung von Strontiumsalzen die Expansionsfähigkeit der aushärtenden Zementpaste eingestellt werden kann. In Versuchen, wie in den Beispielen dargelegt, zeigt sich nämlich, daß die Expansionsfähigkeit der Zementmischung, deren Hauptphase im ausgehärteten Zustand das Magnesium-ammonium-phosphat darstellt, mit zunehmendem Gewichtsanteil von Strontiumsalzen an der Gesamtpulvermischung abnimmt. Demzufolge kann mit dieser Erfindung ein Werkstoff für die Endodontologie zur Verfügung gestellt werden, der neben ausreichender mechanisch belastbaren Stabilität auch eine expandierbare Eigenschaft aufweist.

Gegenstand dieser Erfindung ist es, ein Material für den Knochenersatz, für die Knochenaugmentation und die Knochenregeneration zur Verfügung zu stellen, welches in einer begrenzten Zeit resorbiert werden kann und dessen Druckstabilität den Regenerationserfordemissen des Körpers angepaßt abnehmen kann.
Ebenso ist es Gegenstand dieser Erfindung, ein Material zur Verfügung zu stellen, der bei normalen Temperaturbedingungen, vorzugsweise Körpertemperatur, erstellt, vorbereitet und modelliert werden kann, mit anderen Worten ein Zement.

Charakteristisch für das zur Verfügung gestellte Material ist, dass es zusätzlich durch die Stärke des Sinterungsgrades des eingebrachten Mg₃(PO₄)₂ in seiner Verarbeitungszeit, insbesondere bei Raumtemperatur, eingestellt werden kann, wobei durch den Sinterungsgrad und die daraus resultierende Dichte des verwendeten Mg₃(PO₄)₂ die Lösungsgeschwindigkeit an der Oberfläche dieser Partikel kontrolliert wird, sodaß die zur Erstarrung notwendige Präzipitation der ausfallenden Ca/Mg/Phosphat-Verbindung gesteuert werden kann.

Weiterhin ist es Gegenstand dieser Erfindung, einen Phosphatzement mit partialler Löslichkeit zur Verfügung zu stellen, vorzugsweise durch die langsame Löslichkeit der Magnesium-ammonium-phospat-Zements mit Apatitstrukutr.

Weiterhin ist Gegenstand der vorliegenden Erfindung, einen Reaktionsprozess zu beschreiben, der aus einer Anzahl von Einzelkomponenten, zur Formung eines Magnesium-ammonium-phosphat-Zementes führt und welcher in klinisch akzeptabler Zeit bei Raum- und/oder Körpertemperatur aushärtet.

Weiterhin ist Gegenstand der vorliegenden Erfindung, ein Material zur Verfügung zu stellen, welches in klinisch akzeptabler Zeit ausreichend hart und stabil wird und welches eine starke Adhäsionsfähigkeit an mineralisierten Oberflächen aufweist.

Weiterhin ist Gegenstand der Erfindung, daß sich das nach Art der Erfindung offenbarte Material durch eine starke Adhäsionsfähigkeit an metallischen Oberflächen auszeichnet.

Weiterhin ist Gegenstand der vorliegenden Erfindung, einen resorbierbaren Zement zur Verfügung zu stellen, der im Stadium einer gemischten Paste injizierbar ist.

Ein Aspekt dieser Erfindung ist, daß das Endprodukt aus einer Pulvermischung besteht mit einem molaren Ca/P Verhältnis im Bereich von 1,00 bis 1,50 (P steht für Orthophosphat).

Außerdem ist es essentiell, dass das molare Mg/P Verhältnis dieser Pulvermischung den Bereich von 0 bis 1,00 umfasst.

Um eine Zementpaste zu mischen und zu formen, die innerhalb einer akzeptablen Zeit härtet, müssen diese Pulvermischungen ausreichend reaktiv sein. Um dies zu erreichen, ist ein weiterer Aspekt dieser Erfindung, die Pulvermischungen mit geeigneten Mengen leicht basischer (7<pH<12), wässriger Lösungen aus löslichen ionischen Bestandteilen zu mischen, wie beispielsweise: Na₃PO₄, K₂CO₃, und/oder Na₂CO₃ in Kombination mit (NH₄)₂HPO₄.

Ein weiteres bevorzugtes Merkmal dieser Erfindung ist, daß der aushärtenden Zementpaste granuläre, aber dabei in der Körperflüssigkeit leicht lösliche, granuläre Feststoffe zugemischt werden, so daß nach deren Herauslösüng ein mikro- bis makroporöses Porensystem entsteht.

Ein Vorteil dieser Erfindung ist, dass diese Zemente ihre maximale Festigkeit innerhalb weniger Stunden erreichen.

Ein weiteres bervozugtes Merkmal dieser Erfindung liegt in der Expansionsfähigkeit des Zementes während des Abbindens. Die Expansivität wird durch den relativen Anteil eines zugemischten Strontiumsalzes bestimmt bzw. eingestellt.

Ein weiteres bevorzugtes Merkmal dieser Erfindung ist, daß der ausgehärtete Zement aus mikrokristallinem Magnesium-ammonium-phosphat besteht.

Ein weiteres bevorzugtes Merkmal dieser Erfindung ist, daß die initiale Aushärtezeit des Zementes auf 1 bis 40 Minuten und die finale Aushärtezeit auf 2,5 bis 60 Minuten eingestellt werden kann. (nach ASTM C266-89)

Ein weiteres bevorzugtes Merkmal dieser Erfindung ist, dass der Zement eine maximale Kompressionsfestigkeit von über 50 MPa erreichen kann

Ein weiteres bevorzugtes Merkmal dieser Erfindung ist, dass die Zementpaste vor Erreichen der initialen Aushärtezeit injizierbar ist.

Ein weiteres bevorzugtes Merkmal dieser Erfindung ist, dass die Zementpaste als Trägermaterial für andere Stoffe, die nicht Ca, Mg und/oder Phosphat sind, dienen kann. zum Beispiel, ZnO, pharmazeutische Wirkstoffe (Antibiotika, Zytostatika, Wachstumsfaktoren) oder andere bioaktive Substanzen.

Weitere Merkmale und Zweckmäßigkeiten der Erfindung ergeben sich aus der Beschreibung von Ausführungsbeispielen.

### Beispiele:

In den Beispielen werden folgende Symbole benutzt:
- P =: Pulvermischung
- L =: Flüssigkeit
- L/P =: Flüssigkeit/Pulver Verhältnis in ml/g
- tₗ =: initiale Aushärtezeit (nach ASTM-Norm C266-89, Gilmoore Nadel)
- t_{F} =: finale (end-) Aushärtezeit (nach ASTM-Norm C266-89 Gilmoore Nadel)
- D(x h) =: Kompressionsfestigkeit in Mpa nach x-Stunden Lagerung in 0,9%iger NaCl Lösung bei 37°C

### Herstellung: Nach Einwiegen aller Bestandteile wird die Pulvermischung in einer Kugelmühle für ca. 20 Minuten homogenisiert.

### Beispiel 1:

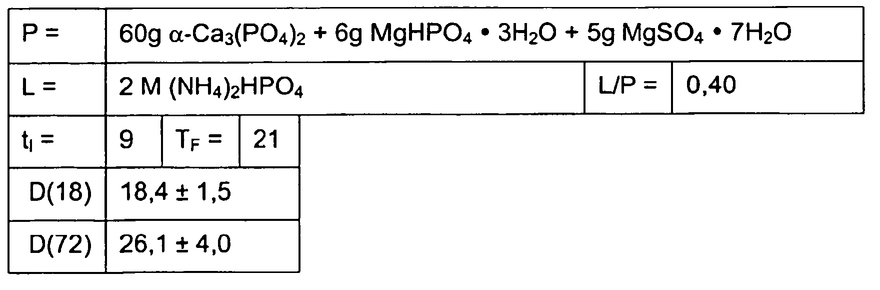

### Beispiel 2:

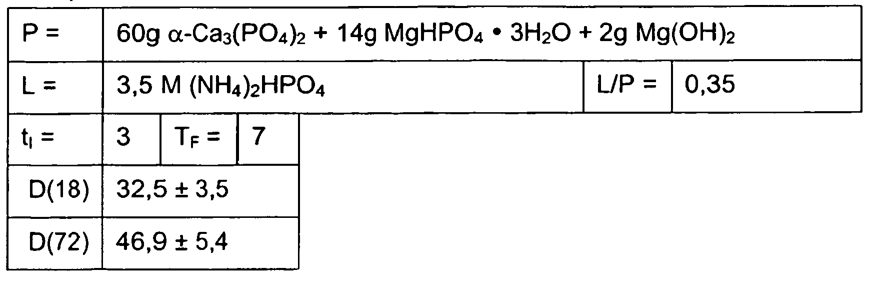

### Beispiel 3:

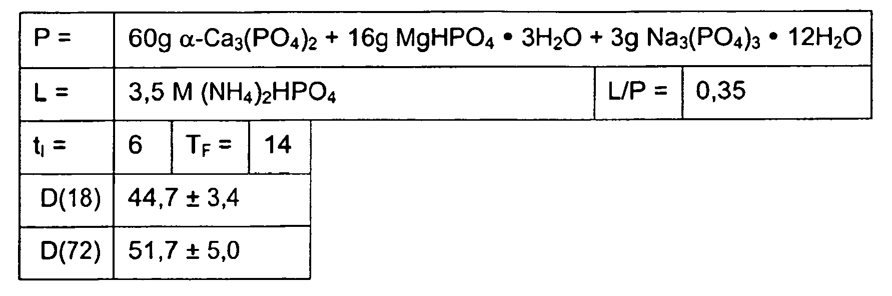

### Beispiel 4:

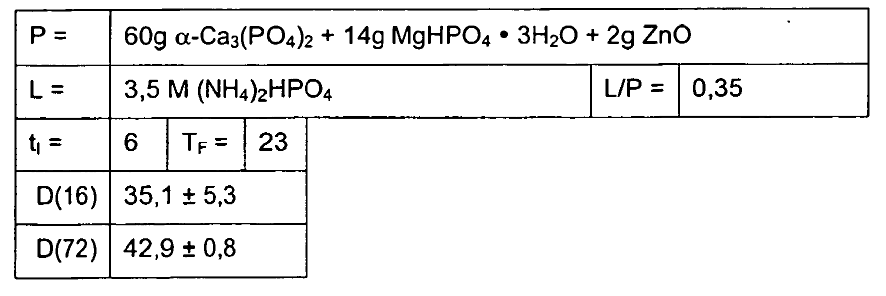

### Beispiel 5:

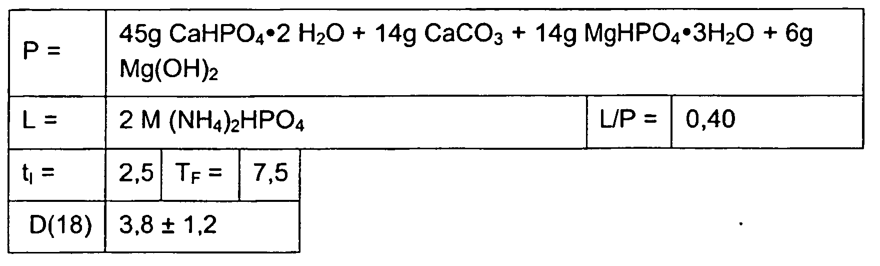

### Beispiel 6:

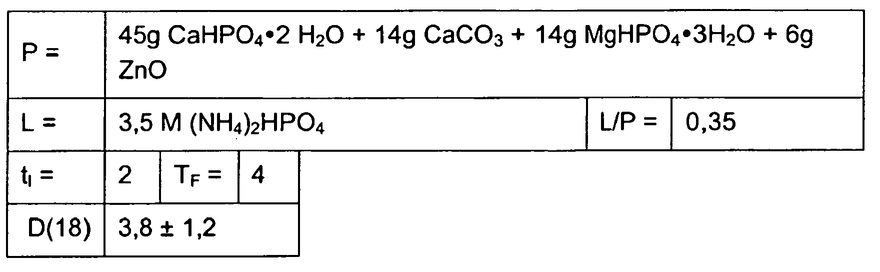

### Beispiel 7:

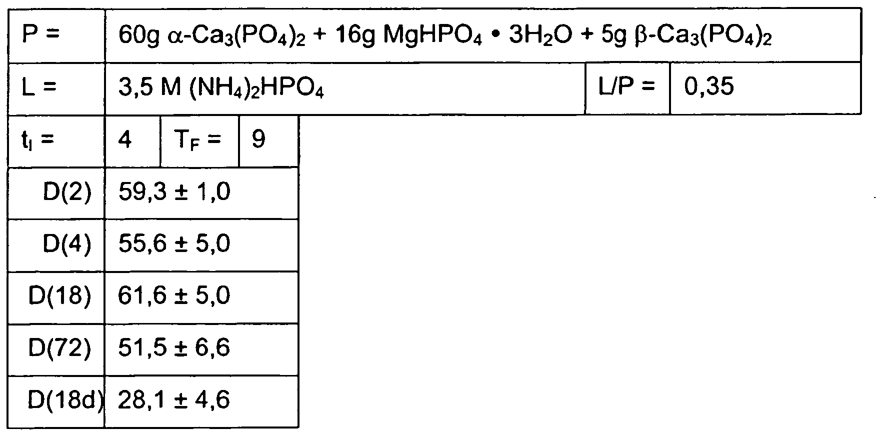

### Beispiel 8:

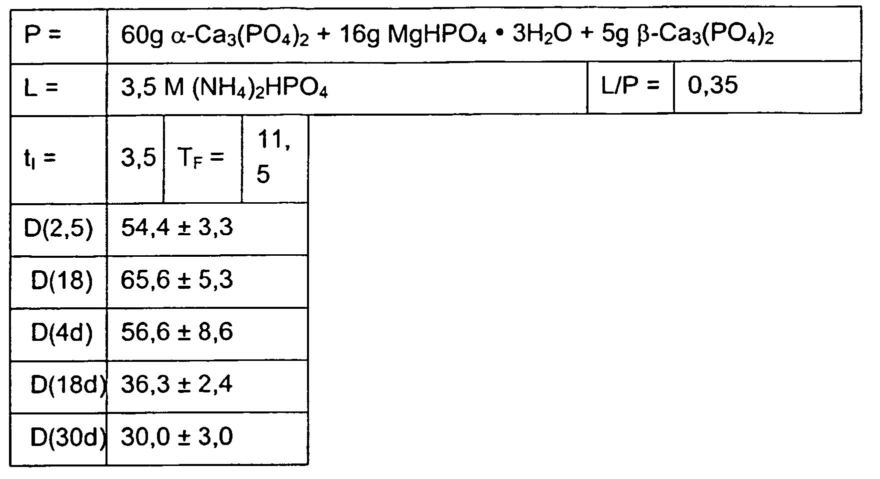

**Beispiel 9:**

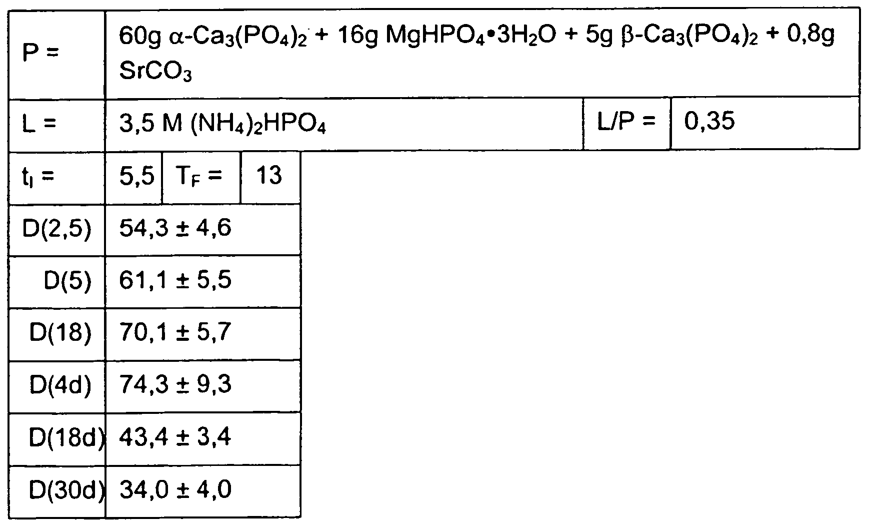

### Beispiel 10:

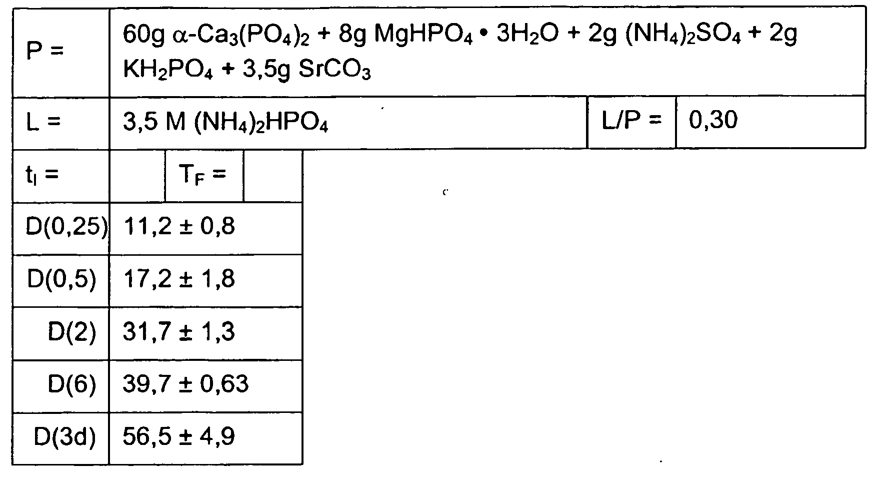

### Beispiel 11:

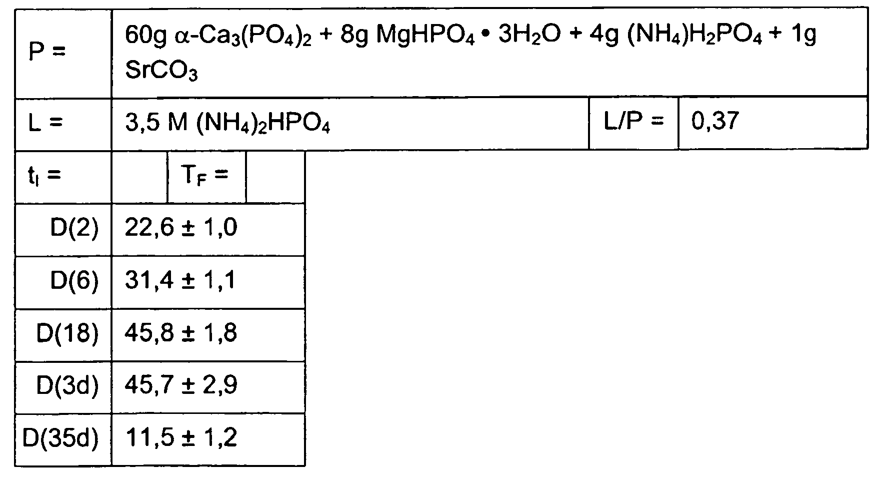

### Beispiel 12:

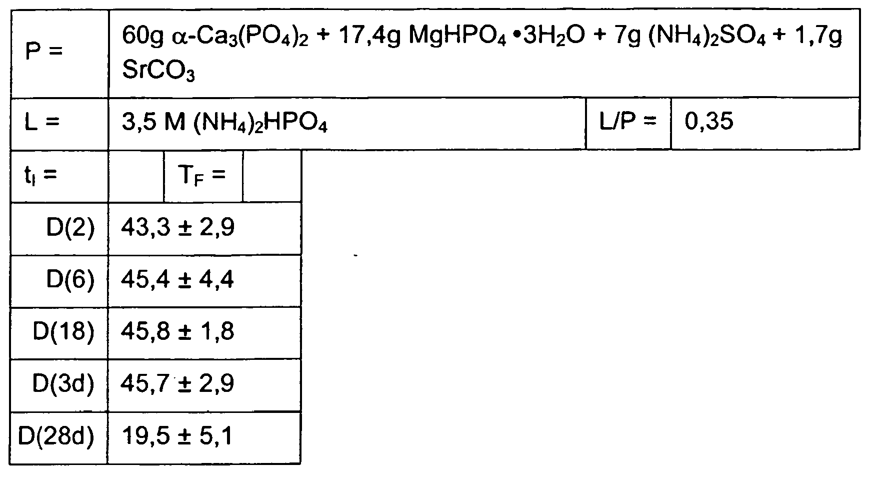

### Beispiel 13:

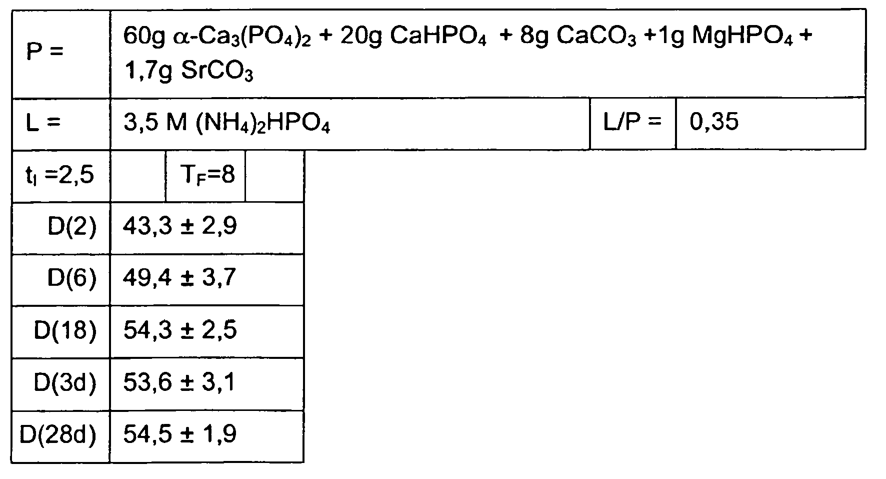

### Beispiel 14:

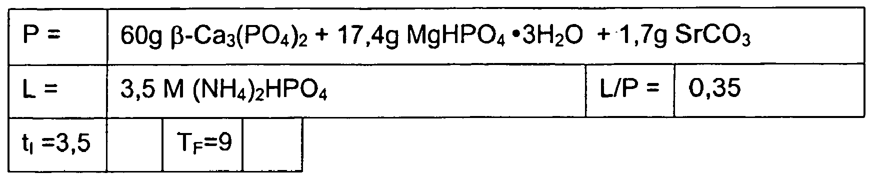

### Beispiel 15:

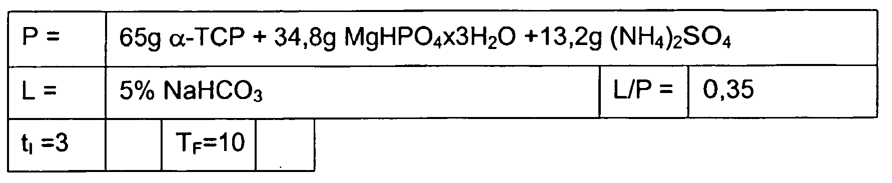

### Beispiel 16:

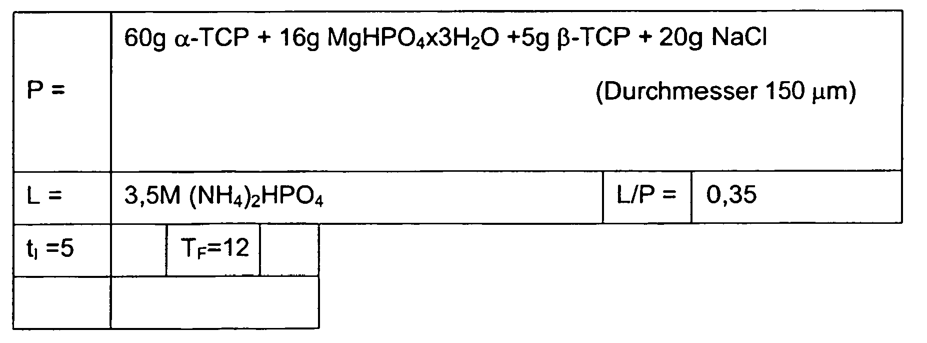

### Beispiel 17:

P= 60g α-TCP + 6g Mg₃(PO₄)₂ + 10g KH₂PO₄ + 5g β-TCP
Anmischlösung: 3,2 molare Lsg. (NH₄)₂HPO₄
UP = 0,35
Tᵢ= 10,8 t_{f}= 20
D(2) = 18,5 ± 1,0
D(18) = 48,3 ± 1,8

## Patentansprüche

1. Magnesium-ammonium-phosphat-Zement-Zubereitung, umfassend
- eine Pulvermischung mit molaren Mengen der Komponenten Calcium (Ca), Magnesium (Mg) und Orthophosphat (P) in der Mischung in den Bereichen 1,00 < Ca/P < 1,50 und 0 < Mg/P < 0,50,
- ein Ammoniumsalz; und
- gegebenenfalls Wasser und /oder eine wäßrige Lösung.

2. Zubereitung nach Anspruch 1, worin die wäßrige Lösung eine wäßrige Lösung eines Ammoniumsalzes mit einem pH-Wert im Bereich von 7 < pH < 12 ist.

3. Zubereitung nach Anspruch 1, worin das Ammoniumsalz in der Pulvermischung enthalten ist und die molaren Mengen der Komponenten Calcium (Ca), Magnesium (Mg), Orthophosphat (P) und Ammonium (NH₄) in den Bereichen 1,00 < Ca/P < 1,50 und 0 < Mg/P < 0,50 und 0 < NH₄ < 0,50 liegen.

4. Zubereitung nach einem der Ansprüche 1 bis 3, worin die Pulvermischung α/β-tertiäres Calciumphosphat (α/β-TCP) und bevorzugt MgHPO₄ x 3 H₂O umfaßt.

5. Zubereitung nach einem der Ansprüche 1 bis 3, wobei die Pulvermischung außer α/β-TCP und MgHPO₄ x 3 H₂O zusätzlich Mg₃(PO₄)₂ enthält.

6. Zubereitung nach einem der Ansprüche 1 bis 5, **dadurch gekennzeichnet, daß** die wäßrige Lösung eine wäßrige (NH₄)₂SO₄-Lösung umfaßt.

7. Zubereitung nach einem der Ansprüche 1 bis 6, worin die Pulvermischung (NH₄)₂SO₄ umfaßt.

8. Zubereitung nach einem der Ansprüche 1 bis 7, worin eine Anmischflüssigkeit aus einer wäßrigen (NH₄)₂HPO₄-Lösung besteht.

9. Zubereitung nach einem der Ansprüche 1 bis 8, worin die Pulvermischung zusätzlich KH₂PO₄ umfaßt.

10. Zubereitung nach einem der Ansprüche 1 bis 9, worin die Pulvermischung zusätzlich Na₂HPO₄ umfaßt.

11. Zubereitung nach einem der Ansprüche 1 bis 10, umfassend zusätzlich SrCO₃.

12. Zubereitung nach Anspruch 11, worin der Gehalt an SrCO₃ 0,01 bis 10 Gew.-% beträgt, vorzugsweise 0,1 bis 5 Gew.-%, bezogen auf das Gesamtgewicht der Zubereitung.

13. Magnesium-ammonium-phosphat-Zement-Zubereitung, umfassend
- eine Pulvermischung, bestehend aus MgHPO₄ x 3 H₂O, KH₂PO₄, Na₂HPO₄, und α-TCP und β-TCP oder α/β-TCP, und ggf. Mg₃(PO₄)₂
- Ammoniumionen; und
- gegebenenfalls Wasser und/oder eine wäßrige Lösung.

14. Zubereitung nach Anspruch 13, umfassend als Ammoniumionen eine wäßrige Lösung eines Ammoniumsalzes in Form einer Anmischflüssigkeit.

15. Magnesium-ammonium-phosphat-Zement-Zubereitung, umfassend
- eine Pulvermischung, bestehend aus α-TCP, β-TCP, KH₂PO₄, Na₂HPO₄ und entweder (NH₄)₂SO₄ oder primärem oder sekundärem Ammoniumphosphat;
- ein Magnesiumsalz, bevorzugt MgHPO₄ x 3 H₂O; und
- gegebenenfalls Wasser und/oder eine wäßrige Lösung.

16. Zubereitung nach Anspruch 15, umfassend als Anmischflüssigkeit eine wäßrige Lösung eines Magnesiumsalzes.

17. Zubereitung nach einem der Ansprüche 13 bis 16, umfassend zusätzlich SrCO₃ in der Pulvermischung, bevorzugt in einer Menge von 0,01 bis 10 Gew.-%, bezogen auf das Gesamtgewicht der Zubereitung.

18. Zubereitung nach einem der Ansprüche 1 bis 17, umfassend als Anmischflüssigkeit eine wäßrige Lösung eines Strontiumsalzes.

19. Zubereitung nach einem der Ansprüche 1 bis 18, umfassend als zusätzliche Komponente ZnO in der Pulvermischung und/oder in der Anmischflüssigkeit.

20. Zubereitung nach einem der Ansprüche 1 bis 19, wobei die Pulvermischung zusätzlich Ca₂NaK(PO₄)₂ und/oder CaKPO₄ enthält.

21. Zubereitung nach einem der Ansprüche 1 bis 20, umfassend als zusätzliche Komponente Fluorid-Ionen in der Pulvermischung und/oder in der Anmischflüssigkeit.

22. Zubereitung nach einem der Ansprüche 1 bis 21, umfassend als zusätzliche Komponenten pharmazeutische und/oder bioaktive Wirkstoffe in der Pulvermischung und/oder in der Anmischflüssigkeit, bevorzugt Antibiotika, Zytostatika, Analgetika, Desinfizienzien, Wachstumsfaktoren, Proteine oder Elastininhibitoren in therapeutischen Dosen.

23. Verfahren zur Herstellung eines Magnesium-ammonium-phosphat-Zements unter Verwendung einer Magnesium-ammonium-Zement-Zubereitung nach einem der Ansprüche 1 bis 22, bei dem die Pulvermischung mit der Anmischflüssigkeit unter Erzielen einer gleichmäßigen Verteilung der Flüssigkeit in der Pulvermischung gemischt wird und die so erhaltene Paste an oder auf der Zielzone aufgetragen oder in die Zielzone eingebracht wird und unter Bildung von mikrokristallinem Magnesium-ammonium-phosphat enthaltendem Zement aushärten gelassen wird.

24. Verfahren nach Anspruch 23, wobei der Pulvermischung granuläre, dabei in wäßrigen Flüssigkeiten leicht lösliche granuläre Partikel, zwischen etwa 10µm und etwa 300µm, bevorzugt zwischen 50µm und 200µm beigefügt werden.

25. Verfahren nach Anspruch 24, wobei die granulären Partikel vorzugsweise aus NaCl, Zuckern, CaSO₄, β-TCP, Polylactiden, Polyglycoliden bzw. Polylactid/Polyglycolid-Copolymer, CaCO₃ CaHPO₄ bestehen.

26. Verwendung einer Zubereitung nach einem der Ansprüche 1 bis 22 zur Herstellung eines Mittels für medizinische Zwecke.

27. Verwendung einer Zubereitung nach einem der Ansprüche 1 bis 22 zur Herstellung eines Zahnzements.

28. Verwendung einer Zubereitung nach einem der Ansprüche 1 bis 22 zur Herstellung eines Knochenersatzes oder Knochenfüllstoffes oder Knochenzementes oder Knochenklebstoffs.

## Claims

1. Magnesium ammonium phosphate cement preparation, comprising
- a powder mixture having molar quantities of the components calcium (Ca), magnesium (Mg) and orthophosphate (P) in the mixture in the ranges 1.00 < Ca/P < 1.50 and 0 < Mg/P < 0.50;
- an ammonium salt; and
- optionally water and/or an aqueous solution.

2. Preparation according to claim 1, wherein the aqueous solution is an aqueous solution of an ammonium salt having a pH value in the range from 7 < pH < 12.

3. Preparation according to claim 1, wherein the ammonium salt is present in the powder mixture and the molar quantities of the components calcium (Ca), magnesium (Mg), orthophosphate (P) and ammonium (NH₄) lie in the ranges 1.00 < Ca/P < 1.50 and 0 < Mg/P < 0.50 and 0 < NH₄ < 0.50.

4. Preparation according to one of claims 1 to 3, wherein the powder mixture comprises α/β-tertiary calcium phosphate (α/β-TCP) and preferably MgHPO₄ x 3 H₂O.

5. Preparation according to one of claims 1 to 3, wherein the powder mixture, apart from α/ß-TCP and MgHPO₄ x 3H₂O, additionally contains Mg₃(PO₄)₂.

6. Preparation according to one of claims 1 to 5, **characterised in that** the aqueous solution comprises an aqueous (NH₄)₂SO₄ solution.

7. Preparation according to one of claims 1 to 6, wherein the powder mixture comprises (NH₄)₂SO₄.

8. Preparation according to one of claims 1 to 7, wherein a mixing liquid consists of an aqueous (NH₄)₂HPO₄ solution.

9. Preparation according to one of claims 1 to 8, wherein the powder mixture additionally comprises KH₂PO₄.

10. Preparation according to one of claims 1 to 9, wherein the powder mixture additionally comprises Na₂HPO₄.

11. Preparation according to one of claims 1 to 10, comprising additionally SrCO₃.

12. Preparation according to claim 11, wherein the content of SrCO₃ is 0.01 to 10 wt.%, preferably 0.1 to 5 wt.%, based on the total weight of the preparation.

13. Magnesium ammonium phosphate cement preparation, comprising
- a powder mixture, consisting of MgHPO₄ x 3 H₂O, KH₂PO₄, Na₂HPO₄, and α-TCP and β-TCP or α/β-TCP, and optionally Mg₃(PO₄)₂;
- an ammonium salt; and
- optionally water and/or an aqueous solution.

14. Preparation according to claim 13, comprising, as ammonium ions, an aqueous solution of an ammonium salt in the form of a mixing liquid.

15. Magnesium ammonium phosphate cement preparation, comprising
- a powder mixture consisting of α-TCP, β-TCP, KH₂PO₄, Na₂HPO₄, and either (NH₄)₂SO₄ or primary or secondary ammonium phosphate;
- a magnesium salt, preferably MgHPO₄ x 3 H₂O; and
- optionally water and/or an aqueous solution.

16. Preparation according to claim 15, comprising an aqueous solution of a magnesium salt as mixing liquid.

17. Preparation according to one of claims 13 to 16 comprising additionally SrCO₃ in the powder mixture, preferably in an amount of 0.01 to 10 wt.%, based on the total weight of the preparation.

18. Preparation according to one of claims 1 to 17, comprising an aqueous solution of a strontium salt as mixing liquid.

19. Preparation according to one of claims 1 to 18, comprising, as additional component, ZnO in the powder mixture and/or in the mixing liquid.

20. Preparation according to one of claims 1 to 19, wherein the powder mixture additionally contains Ca₂NaK(PO₄)₂ and/or CaKPO₄.

21. Preparation according to one of claims 1 to 20, comprising as additional component, fluoride ions in the powder mixture and/or in the mixing liquid.

22. Preparation according to one of claims 1 to 21, comprising, as additional components, pharmaceutical and/or bioactive active ingredients in the powder mixture and/or in the mixing liquid, preferably antibiotics, cytostatic agents, analgesics, disinfectants, growth factors, proteins or elastin inhibitors in therapeutic doses.

23. Process for producing a magnesium ammonium phosphate cement using a magnesium ammonium cement preparation according to one of claims 1 to 22, in which the powder mixture is mixed with the mixing liquid while achieving uniform distribution of the liquid in the powder mixture and the paste thus obtained is applied on or to the target zone or is introduced into the target zone and is allowed to harden with formation of cement containing microcrystalline magnesium ammonium phosphate.

24. Process according to claim 23, wherein granular, thus granular particles which are readily soluble in aqueous liquids, between about 10 µm and about 300 µm, preferably between 50 µm and 200 µm, are added to the powder mixture.

25. Process according to claim 24, wherein the granular particles preferably consist of NaCl, sugars, CaSO₄, β-TCP, polylactides, polyglycolides or polylactide/polyglycolide copolymer, CaCO₃ CaHPO₄.

26. Use of a preparation according to one of claims 1 to 22 for the preparation of an agent for medical purposes.

27. Use of a preparation according to one of claims 1 to 22 for the preparation of a tooth cement.

28. Use of a preparation according to one of claims 1 to 22 for the preparation of a bone replacement or bone filler or bone cement or bone adhesive.

## Revendications

1. Composition de ciment de phosphate de magnésium-ammonium, comprenant :
- un mélange pulvérulent ayant des quantités molaires des composants calcium (Ca), magnésium (Mg) et orthophosphate (P) en mélange dans les gammes 1,00 < Ca/P < 1,50 et 0 < Mg/P < 0,50,
- un sel d'ammonium ; et
- le cas échéant de l'eau et/ou une solution aqueuse.

2. Composition selon la revendication 1, dans laquelle la solution aqueuse est une solution aqueuse d'un sel d'ammonium ayant un pH dans la gamme 7 < pH < 12.

3. Composition selon la revendication 1, dans laquelle le sel d'ammonium est présent dans le mélange pulvérulent et les quantités molaires des composants calcium (Ca), magnésium (Mg), orthophosphate (P) et ammonium (NH₄) se situent dans les gammes 1,00 < Ca/P < 1,50 et 0 < Mg/P < 0,50 et 0 < NH₄ < 0,50.

4. Composition selon l'une des revendications 1 à 3, dans laquelle le mélange pulvérulent comprend du phosphate de calcium α/β-tertiaire (α/β-TCP), et de préférence du MgHPO₄. 3 H₂O.

5. Composition selon l'une des revendications 1 à 3, dans laquelle le mélange pulvérulent comprend, hormis du α/β-TCP et du MgHPO₄.3H₂O, en plus du Mg₃(PO₄)₂.

6. Composition selon l'une des revendications 1 à 5, **caractérisée en ce que** la solution aqueuse comprend une solution aqueuse de (NH₄)₂SO₄.

7. Composition selon l'une des revendications 1 à 6, dans laquelle le mélange pulvérulent comprend du (NH₄)₂SO₄.

8. Composition selon l'une des revendications 1 à 7, dans laquelle un liquide d'addition au mélange se compose d'une solution aqueuse de (NH₄)₂HPO₄.

9. Composition selon l'une des revendications 1 à 8, dans laquelle le mélange pulvérulent comprend en plus du KH₂PO₄.

10. Composition selon l'une des revendications 1 à 9, dans laquelle le mélange pulvérulent comprend en plus du Na₂HPO₄.

11. Composition selon l'une des revendications 1 à 10, comprenant en plus du SrCO₃.

12. Composition selon la revendication 11, dans laquelle la teneur en SrCO₃ est de 0,01 à 10 % en poids, de préférence de 0,1 à 5 % en poids, par rapport au poids total de la composition.

13. Composition de ciment de phosphate de magnésium-ammonium, comprenant :
- un mélange pulvérulent constitué de MgHPO₄.3 H₂O, KH₂PO₄, Na₂HPO₄ et α-TCP et β-TCP ou α/β-TCP, et le cas échéant de Mg₃(PO₄)₂,
- des ions ammonium ; et
- le cas échéant de l'eau et/ou une solution aqueuse.

14. Composition selon la revendication 13, comprenant à titre d'ions ammonium une solution aqueuse d'un sel d'ammonium sous la forme d'un liquide d'addition au mélange.

15. Composition de ciment de phosphate de magnésium - ammonium, comprenant :
- un mélange pulvérulent constitué de α-TCP, β-TCP, KH₂PO₄, Na₂HPO₄ et soit de (NH₄)₂SO₄, soit de phosphate d'ammonium primaire ou secondaire ;
- un sel de magnésium, de préférence MgHPO₄ × 3 H₂O ; et
- le cas échéant de l'eau et/ou une solution aqueuse.

16. Composition selon la revendication 15, comprenant à titre de liquide d'addition au mélange une solution aqueuse d'un sel de magnésium.

17. Composition selon l'une des revendications 13 à 16, comprenant en plus du SrCO₃ dans le mélange pulvérulent, de préférence en une quantité allant de 0,01 à 10 % en poids, par rapport au poids total de la composition.

18. Composition selon l'une des revendications 1 à 17, comprenant à titre de liquide d'addition au mélange une solution aqueuse d'un sel de strontium.

19. Composition selon l'une des revendications 1 à 18, comprenant à titre de composant additionnel du ZnO dans le mélange pulvérulent et/ou dans le liquide d'addition au mélange.

20. Composition selon l'une des revendications 1 à 19, où le mélange pulvérulent comprend en plus du Ca₂NaK(PO₄)₂ et/ou CaKPO₄.

21. Composition selon l'une des revendications 1 à 20, comprenant à titre de composant additionnel des ions fluorures dans le mélange pulvérulent et/ou dans le liquide d'addition au mélange.

22. Composition selon l'une des revendications 1 à 21, comprenant à titre de composants additionnels des principes actifs pharmaceutiques et/ou biologiques dans le mélange pulvérulent et/ou dans le liquide d'addition au mélange, de préférence des antibiotiques, des cytostatiques, des analgésiques, des désinfectants, des facteurs de croissance, des protéines ou des inhibiteurs d'élastine en doses thérapeutiques.

23. Procédé de fabrication d'un ciment de phosphate de magnésium-ammonium en utilisant une composition de ciment de magnésium-ammonium selon l'une des revendications 1 à 22, dans lequel le mélange pulvérulent est mélangé avec le liquide d'addition au mélange en obtenant une répartition homogène du liquide dans le mélange pulvérulent et la pâte ainsi obtenue est déposée sur la zone cible ou est introduite dans la zone cible et est laissée à durcir en formant un ciment contenant du phosphate de magnésium - ammonium microcristallin.

24. Procédé selon la revendication 23, dans lequel le mélange pulvérulent est additionné de particules granulaires, en l'occurrence de particules granulaires légèrement solubles dans les liquides aqueux, entre environ 10 µm et environ 300 µm, de préférence entre 50 µm et 200 µm.

25. Procédé selon la revendication 24, dans lequel les particules granulaires se composent de préférence de NaCl, de sucres, de CaSO₄, de β-TCP, de polylactides, de polyglycolides ou de copolymère de polylactide/ polyglycolide, de CaCO₃, de CaHPO₄.

26. Utilisation d'une composition selon l'une des revendications 1 à 22 pour la fabrication d'un agent destiné à des fins médicales

27. Utilisation d'une composition selon l'une des revendications 1 à 22 pour la fabrication d'un ciment dentaire.

28. Utilisation d'une composition selon l'une des revendications 1 à 22 pour la fabrication d'un implant osseux ou d'une charge osseuse ou d'un ciment osseux ou d'une colle osseuse.
